# EUROPEAN PATENT APPLICATION

(11) **EP 2 865 375 A1**
(43) Date of publication of application: **29.04.2015**
(21) Application number: 14190314.6
(22) Date of filing: 24.10.2014
(51) Int. Cl.: A61K 9/48, A61K 9/70, A23L 1/00

(54) **Coating and extruding method for producing starch softgel capsules**

(30) Priority: 26.10.2013 CN 201310510574
(71) Applicant: Zhongshan Capsule Starch Material Technology Co., Ltd., Guangdong 528451 (CN)
(72) Inventor: SHUAI, Fangwen, 410331 Changsha (CN); WANG, Xiangfeng, 410331 Changsha (CN); ZHANG, Jiawei, 410331 Changsha (CN); ZHANG, Nuozi, 410331 Changsha (CN)
(74) Representative: Clark, Jane Anne

(57) **Abstract**

The invention discloses a method for preparing starch-based softgel capsules, which, specifically, includes the following 2 steps: 1, Extrude the starch-based premixed material into starch-based extrusion film, 2, Apply the gel solution coating to the extrusion film to form composite starch film, 3) Process two composite starch films into starch-based softgel capsules by rotary die process.

## Description

### CROSS REFERENCE TO RELATED APPLICATION

This application claims priority from Chinese Patent Application No. 201310510574.9, filed with State Intellectual Property Office, P. R. C. on October 26, 2013, the entire content of which is incorporated herein by reference.

### FIELD OF INVENTION

This invention relates to a preparation method of softgel capsules, more specifically, of non-gelatin softgel capsules.

### BACKGROUND OF THE INVENTION

Softgel capsules are generally used for enclosing medicines that should not directly contact esophagus and that must be disintegrated in the stomach or intestine. At present, most capsules used for medicine or dietary supplements are made of gelatin extracted from animal skins or bones, and the gelatin extracting process involves acid or alkali treatment, which unavoidably generates animal protein residues. These residues will interact with the enclosed content and thus leads to negative consequences such as drug spoilage.

In addition, animal-derived gelatin is not acceptable to vegetarians and/or the people with Islamic and Jewish beliefs, and those with allergic constitution should also avoid intake of gelatin products.

As starch is made from rich source of plants, its price is far lower than that of gelatin. The starch, when modified, achieves better gelation performance and mechanical strength, making its characteristics close to that of gelatin. Since starch softgel capsules will overcome the shortcomings of existing gelatin capsules and at the same time, has the advantage of low cost, It is worthy of popularization.

Conventionally, the general practice is like this: Apply the prepared gel solution onto the rollers, and then, after the solution becomes glutinous coating due to partially moisture evaporation, send it into the capsule machine for filling, pressing, and forming. In this technology, however, we will have to pre-coat demoulding agent, such as silicone, silicone oil, etc, on the roller and the mold or add such agent to the gel solution formula due to the fact that the coating is adhered to the roller and can hardly be released. This will cause part of the releasing agent enter into the finished softgel capsules and consequently result in unnecessary impurities in the medicine.

On one hand, capsule shell must be quickly dissolved in the stomach and intestines to release active substances and, to store medicine, the capsule shell must be made of weldable material, so as to ensure sufficient stability at the joint seam. On the other hand, if the starch film lacks sufficient elasticity, it will be easily torn, especially in the demoulding process, or lead to final capsule tearing.

An embodiment of the present invention provides an innovative coating-method for preparing starch softgel capsules, the innovation of which lies in that, coating the gel on the starch-based extrusion film----the basic framework of a capsule----, to be more specific, on the side of encapsulation. As the starch extrusion film directly touched to the roller is not adhered to the roller, no releasing agent is needed to separate it from the roller. The softgel capsules prepared using this method remains no residue of releasing agent, and thus significantly improves the safety level.

The invention discloses a preparation method of starch softgel capsules, which includes the following 2 steps:

(a) Composite starch film preparation: Make premixed starch-based material into starch extrusion film by extrusion mechanism, and then apply the gel solution coating to the starch extrusion film to form the composite starch film.

(b) Preparation of starch softgel capsules: Apply two pieces of composite starch films to make starch-based softgel capsules using rotary die process.

### In the above steps:

The preparation method of the starch extrusion film includes the following steps: use double screw-type extruder to extrude the starch based premixed material into starch granules, and then process the starch granules into starch-based extrusion film using single screw-type extruder.

The starch-based premixed material is prefabricated with starch, anti-caking agent, and gelatinization agent

Coating method: Mix water retention agent, gel, and emulsifier in water to prepare the gel solution and then coat this gel solution onto the starch extrusion film to form composite starch film.

### Description of Each Step:

(1) Mix the starch and anti-caking agent for 5 minutes in a high-speed mixer at the speed of 1500-2000rpm and under the temperature of 60 °C, and then slowly add gelatinization into the high-speed mixer while keep stirring for another 5 minutes.

(2) Feed the starch-based premix into a double-screw extruder to make starch granules. Then process the granules into starch-based extrusion films using single screw-type extruder.

(3) Prepare the gel solution by mixing water retention agent and emulsifier in deionized water, and then apply the gel solution coating to the inner side (the encapsulation side) of the starch extrusion film to make composite starch film.

The starch mentioned in the above step 1 is native or modified starch(modified by chemical or physical process), preferably, esterified cassava starch.

The anti-caking agent mentioned in the above step 1 is pharmaceutical stearic acid or

### fatty glyceride

The gelatinization mentioned in the above step 1 is deionized water.

The parameters of the double screw-type extruder mentioned in the above step (2) are as follows: material temperature , 25-160 °C; screw rotation speed, 60-400 rpm; (optimized speed should be 50-150°C, 60-140°C, 70-130°C, 80-140°C, 90-120°C, 100-140°C.)

The parameters of the single screw-type extruder mentioned in the above step (2) are as follows: material temperature, 25-160°C; screw rotation speed, 60-400 rpm.

The gel mentioned in the above step (3) is one or a combination of the substances selected from the group consisting of amylopectin, gellan gum, carrageenan, xanthan gum, guar gum, sodium alginate, and locust bean gum.

The water retention agent mentioned in the above step (3) is pharmaceutical glycerin or pharmaceutical sorbitol.

The emulsifier mentioned in the above step (3) is an ionic emulsifier, preferably pharmaceutical alkali metal sulfate or alkali metal sulfonate, such as pharmaceutical sodium dodecyl sulfonate or pharmaceutical sodium dodecyl sulfate.

The said gel solution consists of the following components:
Gel: 15%-25% of the gel solution (by weight);
Water retaining agent: 1-5 % (by weight); of the gel solution
Emulsifier: 0.02-1% (by weight) of the gel solution;
deionized water.
Thickness of gel solution coating: 0.2-0.5 L gel solution per square meters of starch based extrusion film.

### Preparation of Starch-based softgel capsules

Starch-based softgel capsules are prepared by rotary die process, which is described as follows:

Feed two pieces of composite starch films into the two adjoining cylinder moulds, with the gel-attached extrusion film facing upwards; Adjust the mould temperature to 40-90°C and start the machine. Then, the two cylinder moulds drive the two composite films spinning inward in different direction. The grooves on the mould can be vacuumed to make the composite starch films adhered to the mould form pits, which is then extruded through the mould into softgel capsules with cavity, while at the same time of the capsule formation, the capsule content is filled from right above the junction of the two cylinder moulds.

The starch-based softgel capsules prepared according to this invention can be used for the preparation of drugs, health products, and functional foods.

### EXAMPLES

The examples set forth below further explain the contents of this invention and the nature of products produced using this invention. All the following examples are illustrative, and should not be viewed as limiting the scope of the present invention.

### Example 1

The raw materials used to prepare starch-based extrusion film and their weight ratio:
Starch: corn starch, 75%;
Anti-caking agent: pharmaceutical stearic acid, 1.2%;
Gelatinization agent: Deionized water, 23.8%;

The raw materials used to prepare the gel solution and their weight ratio:
Gel: carrageenan, 25%
Water retaining agent: pharmaceutical glycerin, 1%
Emulsifier: pharmaceutical sodium dodecyl sulfate, 0.2%
Deionized water: 73.8%

The parameters of the double screw-type extruder are as follows

Extrudes at the rotation speed of 350 RPM, and the designed temperature for each slider are as follow
Slide 1: 25°C
Slide 2-3: 110°C
Slide 4-6: 145°C
Slide 7-9: 165°C
Slide 10-12: 160°C
Nozzle: 160°C

The parameters of the single screw-type extruder are as follows

The rotation speed of the screw is 350 RPM, and the designed temperature, 105°C.

The Operation steps are as follows:
(a) Mix the starch and anti-caking agent in the high-speed mixer for 2 minutes at the speed of 1500-2000rpm and under the temperature of 60°C, then slowly adds gelatinization agent into the high-speed mixer while keeps on stirring for another 5 minutes, the starch-based premix is then ready.
(b) Input the starch-based premixed material into the double screw-type extruder to extrude the premixed material into starch granules, and then make the starch granules into starch-based extrusion films using single screw-type extruder.
(c) Prepare the gel solution by dissolving water retention agent and emulsifier in deionized water, and then apply the gel solution coating to the inner side (the encapsulation side) of the starch extrusion film to make composite starch film.

Starch-based capsules preparation: Put the composite starch films into the rotary die with temperature control device to make starch-based capsule. The die temperature is set at 60 °C.

No releasing agent is added during the above preparation processes.

### Example 2

The raw materials used to prepare starch-based extrusion film and their weight ratio:
Starch: starch, 75%;
Anti-caking agent: pharmaceutical stearic acid, 1.2%;
Gelatinization agent: Deionized water, 23.8%;

The raw materials used to prepare the gel solution and their weight ratio:
Gel: cross-linking cassava starch, 25%
Water retaining agent: pharmaceutical glycerin, 1%
Emulsifier: pharmaceutical sodium dodecyl sulfate, 0.2%
Deionized water: 73.8%

Other parameters and processes are the same as example 1.

### Example 3

The raw materials used to prepare starch-based extrusion film and their weight ratio:
Starch: corn starch, 75%;
Anti-caking agent: pharmaceutical stearic acid, 1.2%;
Gelatinization agent: Deionized water, 23.8%;

The raw materials used to prepare the gel solution and their weight ratio:
Gel: guar gum, 25%
Water retaining agent: pharmaceutical sorbitol, 1%
Emulsifier: pharmaceutical sodium dodecyl sulfate, 0.2%
Deionized water: 73.8%

Other parameters and processes are the same as example 1.

### Example 4

The raw materials used to prepare starch-based extrusion film and their weight ratio:
Starch: esterified cassava starch, 75%;
Anti-caking agent: pharmaceutical stearic acid, 1.2%;
Gelatinization agent: Deionized water, 23.8%;

The raw materials used to prepare the gel solution and their weight ratio:
Gel: carrageenan, 20%
Water retaining agent: pharmaceutical glycerin, 3%
Emulsifier: pharmaceutical sodium dodecyl sulfate, 0.2%
Deionized water: 76.8%

Other parameters and processes are the same as example 1.

### Example 5

The raw materials used to prepare starch-based extrusion film and their weight ratio:
Starch: mung bean starch, 75%;
Anti-caking agent: pharmaceutical stearic acid, 1.2%;
Gelatinization agent: Deionized water, 23.8%;

The raw materials used to prepare the gel solution and their weight ratio:
Gel: Xanthan gum, 25%
Water retaining agent: pharmaceutical glycerin, 1%
Emulsifier: pharmaceutical sodium dodecyl sulfate, 0.2%
Deionized water: 73.8%

Other parameters and processes are the same as example 1.

### Example 6

The raw materials used to prepare starch-based extrusion film and their weight ratio:
Starch: corn starch, 75%;
Anti-caking agent: pharmaceutical stearic acid, 2.2%;
Gelatinization agent: Deionized water, 22.8%;

The raw materials used to prepare the gel solution and their weight ratio:
Gel: gellan gum, 25%
Water retaining agent: pharmaceutical sorbitol, 1%
Emulsifier: pharmaceutical sodium dodecyl sulfate, 0.2%
Deionized water: 73.8%

Other parameters and processes are the same as example 1.

### Example 7

The raw materials used to prepare starch-based extrusion film and their weight ratio:
Starch: corn starch, 75%;
Anti-caking agent: pharmaceutical stearic acid, 2.2%;
Gelatinization agent: Deionized water, 22.8%;

The raw materials used to prepare the gel solution and their weight ratio:
Gel: locust bean gum, 25%
Water retaining agent: pharmaceutical sorbitol, 1%
Emulsifier: pharmaceutical sodium dodecyl sulfate, 0.2%
Deionizer water: 73.8%

Other parameters and processes are the same as example 1.

### Comparative Example

The raw material components and their weight ratios are the same as that of example 3; and the preparation processes are as follows:

Put all the above materials into the kettle, and then add deionized water. Heats the water to 60°C and stir it till it is fully dissolved and swelling; Keep the temperature for about 1 to 2 hours till the solution is ready for use. Input the above premixed material to the double screw-type extruder to extrude the premixed material into starch granules, which are then made into starch-based extrusion films using single screw-type extruder.

Starch-based capsules preparation: Put the composite films into the rotary die with temperature control device to make starch-based capsule. The mold temperature is set at 60 °C.

Add regular releasing agent like polyorganosiloxane or methyl silicone oil in the preparation process.

Take 6 samples of capsules. Each sample is made up of certain amount of capsules made by the method applied in one of the 6 examples; inspect the breakage rate and finished product rate of the capsules in each sample. (Refer to Table 1 for the result0 the results in table 1.

Film yield analysis: Demoulding 10000 pieces of films using regular measure, and check the amount of torn and damaged films.

Damage inspection: Put 100 capsules in the friability tester, rotate the tester 100 times, and count the number of damaged capsules.

The above comparative example shows that the performance of the softgel capsules prepared according to this invention is outstanding and complies with the standards of the Chinese Pharmacopoeia. The product made by this invention maintains a relatively high yield rate when no releasing agent is used; and an unexpected finding is that when certain types of gels, like guar gum, xanthan gum, are used, the breakage rate of the product is far lower than when releasing agent is applied. The welding material at the joint seam of the capsule shell directly affects the welding stability. Proper welding material can avoid breakage and leakage resulted from instable welding. The gel solution coating on the starch film framework makes the welding point more stable.

**Table 1**

| | Starch film | | Capsule | |
|---|---|---|---|---|
| | Total broken and damaged pieces | Breakage rate (%) | Broken capsules | Breakage rate |
| Example 1 | 90 | 9.0 | 1 | 1 |
| Example 2 | 91 | 9.1 | 2 | 2 |
| Example 3 | 11 | 1.1 | 0 | 0 |
| Example 4 | 78 | 7.8 | 1 | 1 |
| Example 5 | 15 | 1.5 | 0 | 0 |
| Example 6 | 87 | 8.7 | 2 | 2 |
| Example 7 | 89 | 8.9 | 1 | 1 |
| Comparative example | 93 | 9.3 | 9 | 9 |

According to the inspection regulations described in appendix XA of Chinese Pharmacopoeia 2^{nd}, the disintegration time limit of the capsules made in the above example 1 to 7 is 1 hour.

In an embodiment, the invention discloses a method for preparing starch-based softgel capsules, which, specifically, includes the following 2 steps: 1, Extrude the starch-based premixed material into starch-based extrusion film, 2, Apply the gel solution coating to the extrusion film to form composite starch film, 3) Process two composite starch films into starch-based softgel capsules by rotary die process.

## Claims

1. A method for preparing starch-based softgel capsules comprising:
(A) make the starch-based premixed material into starch-based extrusion film through extrusion mechanism, and then apply gel solution coating onto this extrusion film to form composite film; and
(B) preparation of starch-based softgel capsules comprising process two pieces of composite starch films into starch-based softgel capsules by rotary die process.

2. The method according to claim 1, wherein the film-forming method of the said starch-based extrusion film comprises (a) feed the starch-based premix into the double- screw extruder, through which, the premix is processed into starch granules; and (b) process the starch granules into starch-based extrusion film through the single-screw extruder.

3. The method according to claim 2, wherein said starch-based premixed material is prepared by mixing the starch, anti-caking agent, and gelatinization agent, among which, the said starch is native or modified starch modified by chemical or physical processes; the anti-caking agent, pharmaceutical stearic acid or glycerol esters of fatty acids; and the gelatinization agent, DI water.

4. The method according to claim 1, 2 or 3, wherein said coating method comprises 1) dissolve water retention agent and emulsifier in water to form the gel solution; and 2) apply the gel solution coating to the starch-based extrusion film to form composite starch film.

5. The method according to claim 4, wherein the amount of said gel solution coated on the starch-based extrusion film is 0.2-0.5 liter per square meter starch-based extrusion film.

6. The method according to claim 4 or 5, wherein the said gel is one or a combination of the substances selected from the group consisting of amylopectin, gellan gum, carrageenan, xanthan gum, guar gum , sodium alginate, and locust bean gum; the water retention agent is polyhydric fatty alcohol; and the emulsifier is ionic emulsifier.

7. The method according to claim 4, 5 or 6, wherein said gel solution wherein said gel solution comprises:
water retaining agent at 1-5% by weight of the gel solution;
emulsifier at 0.02-1% by weight of the gel solution; and
deionized water.

8. The use of the starch-based softgel capsules in the fields of medicines, health products, and function foods prepared using the method according to any preceding claim.
